# EUROPÄISCHE PATENTANMELDUNG

(11) **EP 2 540 712 A1**
(43) Veröffentlichungstag der Anmeldung: **02.01.2013**
(21) Anmeldenummer: 11172082.7
(22) Anmeldetag: 30.06.2011
(51) Int. Cl.: C07D 307/28, C07D 307/93, C07D 311/94

(54) **Verfahren zu Herstellung cyclischer Enolether**

(71) Anmelder: BASF SE, 67056 Ludwigshafen (DE)
(72) Erfinder: Ebel, Klaus, 68623 Lampertheim (DE); Brunner, Bernhard, 69120 Heidelberg (DE); Stock, Christoph, 67158 Ellerstadt (DE); Pelzer, Ralf, 37699 Fürstenberg (DE)

(57) **Zusammenfassung**

Die vorliegende Erfindung betrifft ein Verfahren zur Herstellung von cyclischen Enolethern der Formeln (I) und/oder (II).

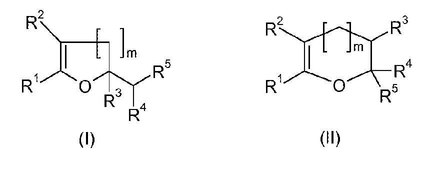

## Beschreibung

Die vorliegende Erfindung betrifft ein Verfahren zur Herstellung von cyclischen Enolethern.

Cyclische Enolether stellen bei der Synthese von makrocyclischen Lactonen, die als Riechstoffe verwendet werden, wichtige Zwischenprodukte dar. So wird in US 3,890,353 die Herstellung des gesättigten 15-Pentadecanolid (Exaltolide^{®}) der Formel (a) beschrieben, 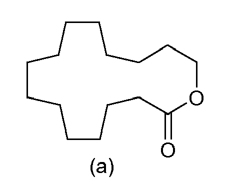 wobei als Ausgangsprodukt der cyclische Enolether 13-Oxa-1.12-didehydro-bicyclo[10.4.0] hexadecan der Formel (b) dient.

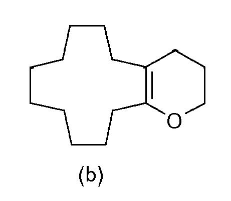

Neben den voran beschriebenen 16-Ring Lactonen sind auch 15-Ring Lactone als moschus-ähnliche Riechstoffe beschrieben. So werden in EP 0 862 911 gesättigte und ungesättigte 15-Ring Lactone der Formeln (d1) und (d2) beschrieben, wobei R gleich Methyl oder Wasserstoff ist.

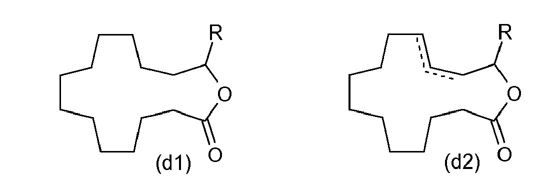

Die 15-Ring Lactone können ausgehend vom entsprechenden cyclischen Enolether der Formel (e) hergestellt werden. 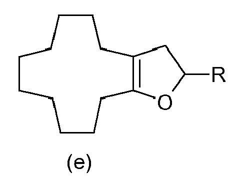 mit R gleich H oder Me

Die Herstellung von cyclischen Enolethern, die als Riechstoffe oder zur Herstellung von Riechstoffen geeignet sind, wird beispielsweise in GB 1266092, DE 2136496, US 3,890,353, DE 25 11 410, DE 29 06 296 oder JP 2010-95447 beschrieben.

Teils sind die Ausgangsprodukte zur Herstellung der cyclischen Enolether nicht einfach zugänglich beziehungsweise die Herstellung der cyclischen Enolether verläuft über mehrstufige Synthesen mit zum Teil schlechten Ausbeuten, teils sind die Reaktionsbedingungen für die Cyclisierung recht drastisch, beispielsweise durch Verwendung größerer Mengen konzentrierter Schwefelsäure, oder die Aufarbeitung der cyclischen Enolether ist in wirtschaftlicher Sicht noch nicht zufrieden stellend oder es werden große Überschüsse von Reagenzien oder Ausgangsstoffen benötigt.

Ausgehend von diesem Stand der Technik bestand die Ausgabe darin, flexible und effizientere Synthesewege zu solchen Riechstoffen zu finden.

Diese Aufgabe wird durch ein Verfahren zur Herstellung von cyclischen Enolethern der Formeln (I) und/oder (II) 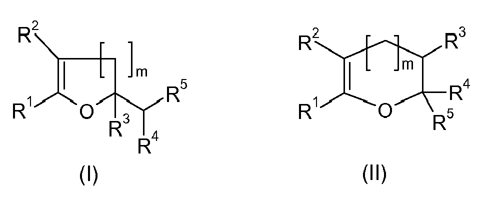 durch Cyclisierung einer Ausgangsverbindung der Formel (III) 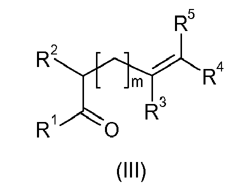 worin
- m: gleich null (0), eins (1) oder zwei (2) ist,
- R¹: ein organischer Rest mit 1 bis 20 Kohlenstoffatomen ist,
- R²: Wasserstoff oder ein organischer Rest mit 1 bis 20 Kohlenstoffatomen ist,
oder die Reste R¹ und R² zusammen mit den sie verbindenden Atomen ein mono- oder polycyclisches, substituiertes oder unsubstituiertes Ringsystem mit 3 bis 20 Kohlenstoffatomen bilden, das auch Heteroatome, ausgewählt aus der Gruppe bestehend aus den Elementen Si, N, P, O, und S enthalten kann,
- R³: Wasserstoff oder ein organischer Rest mit 1 bis 20 Kohlenstoffatomen ist,
- R⁴: Wasserstoff oder ein organischer Rest mit 1 bis 20 Kohlenstoffatomen ist, und
- R⁵: Wasserstoff oder ein organischer Rest mit 1 bis 20 Kohlenstoffatomen ist,

in Gegenwart einer Brönstedt- oder Lewis-Säure, wobei die Reaktion als Reak-tivdestillation durchgeführt wird, wobei die gebildeten cyclischen Enolether der Formeln (I) und/oder (II) destillativ aus dem Reaktionsgemisch von der Ausgangsverbindung der Formel (III) abgetrennt werden, gelöst.

Bei den im erfindungsgemäßen Verfahren verwendbaren Brönstedt-Säuren handelt es sich sowohl um organische als auch um anorganische Säuren. Bevorzugt werden Brönstedt-Säuren, die selbst nicht mit der Ausgangsverbindung der Formel (III) reagieren können, das heißt in einer Reaktion verbraucht werden, sondern lediglich als Protonenquelle für eine durch Protonen katalysierte chemische Reaktion dienen. Nicht einschränkende Beispiele für besonders geeignete Brönstedt-Säuren sind Schwefelsäure, Phosphorsäure, Methansulfonsäure, p-Toluolsulfonsäure, starke saure lonenaustauscher, Tetrafluoroborsäure, Trifluoressigsäure, Ameisensäure oder Oxalsäure.

Als Lewis-Säure können in dem erfindungsgemäßen Verfahren beispielsweise Aluminiumtrichlorid, Zinntetrachlorid, Titantetrachlorid, Zirkoniumtetrachlorid, Eisentrichlorid oder Nickeldichlorid eingesetzt werden.

Die Menge der Brönstedt- oder Lewis-Säure, die im erfindungsgemäßen Verfahren verwendet wird, kann in einem breiten Bereich variiert werden. Prinzipiell kann das molare Verhältnis der Brönstedt- oder Lewis-Säure zur Verbindung der Formel (III) größer, gleich oder kleiner als 1 sein. Prinzipiell reichen Spuren von Säure, um die Cyclisierung zu katalysieren.

Bevorzugt ist in dem erfindungsgemäßen Verfahren das molare Verhältnis der Brönstedt- oder Lewis-Säure, insbesondere der Brönstedt-Säure zur Verbindung der Formel (III) nicht größer als 1, besonders bevorzugt nicht größer als 0,15, ganz besonders bevorzugt zwischen 0,1 und 0,0005, insbesondere zwischen 0,07 und 0,001.

Bevorzugt wird in dem erfindungsgemäßen Verfahren die Cyclisierung in Gegenwart einer Brönstedtsäure durchgeführt. Bevorzugt werden dabei Brönstedtsäuren mit einem pKₛ-Wert von kleiner 5, besonders bevorzugt kleiner als 2,5, insbesondere kleiner als 0 eingesetzt. Ganz besonders bevorzugt liegt der pKₛ-Wert der Brönstedtsäure zwischen -1,5 und -11.

Eine Reaktivdestillation ist ein dem Fachmann prinzipiell bekanntes chemisches Verfahren, bei dem eine ein- oder mehrstufige Destillation mit einer chemischen Reaktion, im vorliegenden Fall einer Cyclisierung, verbunden wird. Das Reaktionsprodukt, im vorliegenden Fall ein cyclischer Enolether der Formeln (I) und/oder (II), wird laufend durch Destillation vom Ausgangsprodukt, einem Keton, abgetrennt.

Bevorzugt wird die Reaktivdestillation bzw. die Cyclisierungsreaktion in einem Temperaturbereich zwischen 50 °C und 300 °C, besonders bevorzugt zwischen 80 °C und 200 °C durchgeführt.

In Abhängigkeit von den Siedepunkten der zu trennenden Verbindungen kann der Fachmann üblicherweise unmittelbar oder nach wenigen Versuchen bezüglich der verwendbaren Destillationskolonnen, dem notwendigen Trennleistungsvermögen einer solchen Kolonne sowie den Destillationsparametern wie beispielsweise Druck, Temperatur und Rücklaufverhältnis geeignete Maßnahmen ergreifen, beziehungsweise eine geeignete Auswahl treffen, um das erfindungsgemäße Verfahren in der gewünschten Weise durchführen zu können.

Die Substituenten gemäß der vorliegenden Erfindung sind, soweit nicht weiter eingeschränkt, wie folgt definiert:
Der Begriff "organischer Rest mit 1 bis 20 Kohlenstoffatomen", wie vorangehend verwendet, bezeichnet beispielsweise C₁-C₂₀-Alkylreste, gesättigte C₃-C₂₀-heterocyclische Reste, C₆-C₂₀-Arylreste, C₂-C₂₀-heteroaromatische Reste oder C₇-C₂₀-Arylalkylreste, wobei der kohlenstoffhaltige Rest weitere Heteroatome ausgewählt aus der Gruppe der Elemente bestehend aus F, Cl, Br, I, N, P, Si, O und S enthalten kann und/oder mit funktionellen Gruppen substituiert sein kann.
Der Begriff "Alkyl", wie vorliegend verwendet, beinhaltet lineare oder ein- bzw. ggf. auch mehrfach verzweigte gesättigte Kohlenwasserstoffe, die auch cyclisch sein können. Bevorzugt ist ein C₁-C₁₀-Alkyl, wie Methyl, Ethyl, n-Propyl, n-Butyl, n-Pentyl, n-Hexyl, n-Heptyl, n-Octyl, n-Nonyl, n-Decyl, Cyclopentyl, Cyclohexyl, Isopropyl, Isobutyl, Isopentyl, Isohexyl, sec-Butyl oder tert-Butyl.
Der Begriff "gesättigter heterocyclischer Rest", wie vorangehend verwendet, bezeichnet beispielsweise mono- oder polycyclische, substituierte oder unsubstituierte Kohlenwasserstoffreste, in denen ein oder mehrere Kohlenstoffatome, CH-Gruppen und / oder CH₂-Gruppen durch Heteroatome, vorzugsweise ausgewählt aus der Gruppe bestehend aus den Elementen O, S, N und P, ersetzt sind. Bevorzugte Beispiele für substituierte oder unsubstituierte gesättigte heterocyclische Reste sind Pyrrolidinyl, Imidazolidinyl, Pyrazolidinyl, Piperidyl, Piperazinyl, Morpholinyl, Tetrahydrofuranyl, Tetrahydropyranyl, Tetrahydrothiophenyl und dergleichen, sowie mit Methyl-, Ethyl-, Propyl-, Isopropyl- und tert-Butylresten substituierte Derivate davon.
Der Begriff "Aryl", wie vorangehend verwendet, bezeichnet beispielsweise aromatische und gegebenenfalls auch kondensierte polyaromatische Kohlenwasserstoffreste, die gegebenenfalls mit linearem oder verzweigtem C₁-C₁₀-Alkyl, C₁-C₁₀-Alkoxy, C₂-C₁₀-Alkenyl oder Halogen, insbesondere Fluor, ein- oder mehrfach substituiert sein können. Bevorzugte Beispiele für substituierte und unsubstituierte Arylreste sind insbesondere Phenyl, Fluorphenyl, 4-Methylphenyl, 4-Ethylphenyl, 4-n-Propylphenyl, 4-Isopropylphenyl, 4-tert-Butylphenyl, 4-Methoxyphenyl, 1-Naphthyl, 9-Antryl, 9-Phenantryl, 3,5-Dimethylphenyl, 3,5-Di-tert-butylphenyl oder 4-Trifluormethylphenyl.
Der Begriff "heteroaromatischer Rest", wie vorangehend verwendet, bezeichnet beispielsweise aromatische Kohlenwasserstoffe, in denen ein oder mehrere Kohlenstoffatome durch Stickstoff-, Phosphor-, Sauerstoff- oder Schwefelatome oder Kombinationen davon ersetzt sind. Diese können wie die Arylreste gegebenenfalls mit linearem oder verzweigtem C₁-C₁₀-Alkyl, C₁-C₁₀-Alkoxy, C₂-C₁₀-Alkenyl oder Halogen, insbesondere Fluor, ein- oder mehrfach substituiert sein. Bevorzugte Beispiele sind Furyl, Thienyl, Pyrrolyl, Pyridyl, Pyrazolyl, Imidazolyl, Oxazolyl, Thiazolyl, Pyrimidinyl, Pyrazinyl und dergleichen, sowie mit Methyl-, Ethyl-, Propyl-, Isopropyl- und tert-Butylresten substituierte Derivate davon.
Der Begriff "Arylalkyl", wie vorangehend verwendet, bezeichnet beispielsweise arylhaltige Substituenten, deren Arylrest über eine Alkylkette mit dem entsprechenden Rest des Moleküls verknüpft ist. Beispiele sind Benzyl, substituiertes Benzyl, Phenethyl, substituiertes Phenethyl und dergleichen.

Der Rest R¹ ist ein organischer Rest mit 1 bis 20 Kohlenstoffatomen, wie beispielsweise C₁-C₂₀-Alkyl, C₆-C₂₀-Aryl, Arylalkyl oder Alkylaryl mit 1 bis 10, vorzugsweise 1 bis 4 Kohlenstoffatomen im Alkylrest und 6 bis 14, vorzugsweise 6 bis 10, insbesondere 6 Kohlenstoffatomen im Arylrest, ein gesättigter heterocyclischer Rest mit 3 bis 20 Kohlenstoffatomen oder ein heteroaromatischer Rest mit 3 bis 20 Kohlenstoffatomen mit jeweils mindestens einem Heteroatom ausgewählt aus der Gruppe bestehend aus den Elementen N, P O und S, insbesondere N, O und S, wobei der heteroaromatische Rest mit weiteren Resten R¹⁰ substituiert sein kann, wobei R¹⁰ ein organischer Rest mit 1 bis 10, insbesondere 1 bis 6 Kohlenstoffatomen ist, wie beispielsweise C₁-C₄-Alkyl, C₆-C₁₀-Aryl, Arylalkyl oder Alkylaryl mit 1 bis 4 Kohlenstoffatomen im Alkylrest und 6 bis 10, vorzugsweise 6 Kohlenstoffatomen im Arylrest, und mehrere Reste R¹⁰ gleich oder verschieden sein können.

Bevorzugt ist R¹ ein linearer C₁-C₁₀-, insbesondere C₁-C₄-Alkylrest, ein verzweigter C₃-C₁₀-, insbesondere C₃-C₄-Alkylrest, ein C₄-C₁₀-, insbesondere C₅-C₈-Cycloalkylrest. Besonders bevorzugt ist R¹ ein linearer C₁-C₁₀-, insbesondere C₁-C₄-Alkylrest.

Der Rest R² ist Wasserstoff oder ein organischer Rest mit 1 bis 20 Kohlenstoffatomen, wie beispielsweise C₁-C₂₀-Alkyl, C₆-C₂₀-Aryl, Arylalkyl oder Alkylaryl mit 1 bis 10, vorzugsweise 1 bis 4 Kohlenstoffatomen im Alkylrest und 6 bis 14, vorzugsweise 6 bis 10, insbesondere 6 Kohlenstoffatomen im Arylrest, ein gesättigter heterocyclischer Rest mit 3 bis 20 Kohlenstoffatomen oder ein heteroaromatischer Rest mit 3 bis 20 Kohlenstoffatomen mit jeweils mindestens einem Heteroatom ausgewählt aus der Gruppe bestehend aus den Elementen N, P O und S, insbesondere N, O und S, wobei der heteroaromatische Rest mit weiteren Resten R¹⁰ substituiert sein kann.

Bevorzugt ist R² ein linearer C₁-C₁₀-, insbesondere C₁-C₄-Alkylrest, ein verzweigter C₃-C₁₀-, insbesondere C₃-C₄-Alkylrest, ein C₄-C₁₀-, insbesondere C₅-C₈-Cycloalkylrest. Besonders bevorzugt ist R² ein linearer C₁-C₁₀-, insbesondere C₁-C₄-Alkylrest.

Alternativ bilden die Reste R¹ und R² zusammen mit den sie verbindenden Atomen ein mono-oder polycyclisches, substituiertes oder unsubstituiertes Ringsystem mit 3 bis 20, bevorzugt 5 bis 14 Kohlenstoffatomen, das auch Heteroatome, ausgewählt aus der Gruppe bestehend aus den Elementen Si, N, P, O, und S enthalten kann. Bevorzugt stehen die Reste R¹ und R² zusammen für eine zweibindige Gruppe -(CH₂)ₓ-, wobei x eine ganze Zahl von 3 bis 12, bevorzugt 4 bis 12, insbesondere 10 bedeutet.

Der Rest R³ ist Wasserstoff oder ein organischer Rest mit 1 bis 20 Kohlenstoffatomen, wie beispielsweise C₁-C₂₀-Alkyl, C₆-C₂₀-Aryl, Arylalkyl oder Alkylaryl mit 1 bis 10, vorzugsweise 1 bis 4 Kohlenstoffatomen im Alkylrest und 6 bis 14, vorzugsweise 6 bis 10, insbesondere 6 Kohlenstoffatomen im Arylrest, ein gesättigter heterocyclischer Rest mit 3 bis 20 Kohlenstoffatomen oder ein heteroaromatischer Rest mit 3 bis 20 Kohlenstoffatomen mit jeweils mindestens einem Heteroatom ausgewählt aus der Gruppe bestehend aus den Elementen N, P O und S, insbesondere N, O und S, wobei der heteroaromatische Rest mit weiteren Resten R¹⁰ substituiert sein kann.

Bevorzugt ist R³ Wasserstoff, ein linearer C₁-C₁₀-, insbesondere C₁-C₄-Alkylrest, ein verzweigter C₃-C₁₀-, insbesondere C₃-C₄-Alkylrest, ein C₄-C₁₀-, insbesondere C₅-C₈-Cycloalkylrest. Besonders bevorzugt ist R³ Wasserstoff oder Methyl.

Der Rest R⁴ ist Wasserstoff oder ein organischer Rest mit 1 bis 20 Kohlenstoffatomen, wie beispielsweise C₁-C₂₀-Alkyl, C₆-C₂₀-Aryl, Arylalkyl oder Alkylaryl mit 1 bis 10, vorzugsweise 1 bis 4 Kohlenstoffatomen im Alkylrest und 6 bis 14, vorzugsweise 6 bis 10, insbesondere 6 Kohlenstoffatomen im Arylrest, ein gesättigter heterocyclischer Rest mit 3 bis 20 Kohlenstoffatomen oder ein heteroaromatischer Rest mit 3 bis 20 Kohlenstoffatomen mit jeweils mindestens einem Heteroatom ausgewählt aus der Gruppe bestehend aus den Elementen N, P O und S, insbesondere N, O und S, wobei der heteroaromatische Rest mit weiteren Resten R¹⁰ substituiert sein kann.

Bevorzugt ist R⁴ Wasserstoff, ein linearer C₁-C₁₀-, insbesondere C₁-C₄-Alkylrest, ein verzweigter C₃-C₁₀-, insbesondere C₃-C₄-Alkylrest, ein C₄-C₁₀-, insbesondere C₅-C₈-Cycloalkylrest. Besonders bevorzugt ist R⁴ Wasserstoff oder Methyl.

Der Rest R⁵ ist Wasserstoff oder ein organischer Rest mit 1 bis 20 Kohlenstoffatomen, wie beispielsweise C₁-C₂₀-Alkyl, C₆-C₂₀-Aryl, Arylalkyl oder Alkylaryl mit 1 bis 10, vorzugsweise 1 bis 4 Kohlenstoffatomen im Alkylrest und 6 bis 14, vorzugsweise 6 bis 10, insbesondere 6 Kohlenstoffatomen im Arylrest, ein gesättigter heterocyclischer Rest mit 3 bis 20 Kohlenstoffatomen oder ein heteroaromatischer Rest mit 3 bis 20 Kohlenstoffatomen mit jeweils mindestens einem Heteroatom ausgewählt aus der Gruppe bestehend aus den Elementen N, P O und S, insbesondere N, O und S, wobei der heteroaromatische Rest mit weiteren Resten R¹⁰ substituiert sein kann.

Bevorzugt ist R⁵ Wasserstoff, ein linearer C₁-C₁₀-, insbesondere C₁-C₄-Alkylrest, ein verzweigter C₃-C₁₀-, insbesondere C₃-C₄-Alkylrest, ein C₄-C₁₀-, insbesondere C₅-C₈-Cycloalkylrest. Besonders bevorzugt ist R⁵ Wasserstoff oder Methyl.

Der Indice m ist gleich null (0), eins (1) oder zwei (2). Im Falle von Verbindungen der Formel (1) ist m bevorzugt eins (1) oder zwei (2) und im Falle von Verbindungen der Formel (II) ist m bevorzugt null (0) oder eins (1). Ganz besonders bevorzugt ist m gleich eins (1) oder zwei (2), insbesondere gleich eins (1).

Die Bildung entweder des cyclischen Enolethers der Formel (I) und/oder der Formel (II) ausgehend von einer bestimmten Ausgangsverbindung der Formel (III) hängt maßgeblich davon ab, an welchem Kohlenstoffatom der Doppelbindung in der Ausgangsverbindung, entweder das den Rest R³ oder das die Reste R⁴ und R⁵ tragende Kohlenstoffatom, sich durch Protonierung der Doppelbindung formal das stabilere Carbokation ausbilden kann. Beispielsweise bilden sich in den Fällen, in denen m gleich 1, R³ gleich Methyl und R⁴ sowie R⁵ gleich Wasserstoff sind, cyclische Enolether der Formel (I) aus, und in den Fällen, in denen m gleich 1, R³ gleich Wasserstoff und R⁴ sowie R⁵ gleich Methyl sind, bilden sich cyclische Enolether der Formel (II) aus.

Bevorzugt sind in dem erfindungsgemäßen Verfahren die Indices in den Formeln (I), (II) und (III) wie folgt definiert.

Der Rest R¹ ist ein C₁-C₁₀- bevorzugt C₁-C₄-Alkylrest, bevorzugt ein linearer C₁-C₁₀- bevorzugt C₁-C₄-Alkylrest.

Der Rest R² ist ein C₁-C₁₀- bevorzugt C₁-C₄-Alkylrest, bevorzugt ein linearer C₁-C₁₀- bevorzugt C₁-C₄-Alkylrest.

Alternativ stehen die Reste R¹ und R² zusammen für eine zweibindige Gruppe -(CH₂)ₓ-, wobei x eine ganze Zahl von 3 bis 12, bevorzugt 4 bis 12, insbesondere 10 bedeutet.

Der Rest R³ ist Wasserstoff oder Methyl.

Der Rest R⁴ ist Wasserstoff oder Methyl.

Der Rest R⁵ ist Wasserstoff oder Methyl.

Der Indice m ist gleich null (0), eins (1) oder zwei (2). Im Falle von Verbindungen der Formel (I) ist m bevorzugt eins (1) oder zwei (2) und im Falle von Verbindungen der Formel (II) ist m bevorzugt null (0) oder eins (1). Ganz besonders bevorzugt ist m gleich eins (1) oder zwei (2), insbesondere gleich eins (1).

Ebenfalls bevorzugt ist ein erfindungsgemäßes Verfahren wie vorangehend beschrieben, wobei die Ausgangsverbindung der Formel (III), die für eine Verbindung der Formel (IIIa) oder eine Verbindung der Formel (IIIb) steht, 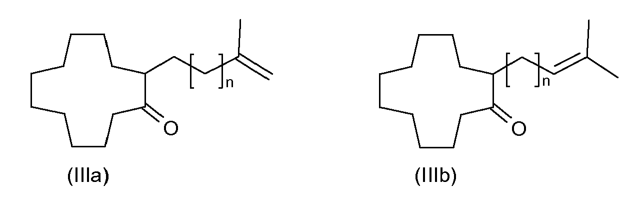 zu dem entsprechenden cyclischen Enolether der Formel (IIa) umgesetzt wird, 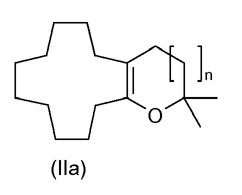 worin in den Formeln (IIa), (IIIa) und (IIIb)
- n: gleich null (0) oder eins (1) ist.

Besonders bevorzugt ist in Verbindungen der Formel (IIIa) n gleich null (0), was zur Bildung des 5-gliedrigen Cycloenolethers führt, und in Verbindungen der Formeln (IIIb) ist n gleich 1, was zur Bildung des 6-gliedrigen Cycloenolethers führt.

Die Erfindung wird durch folgende, die Erfindung jedoch nicht einschränkende Beispiele erläutert.

### Beispiel 1:

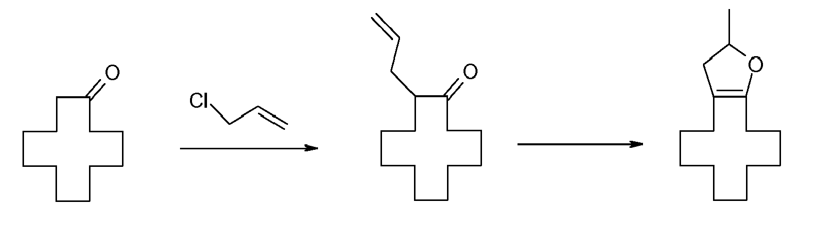

### Allyllierung:

CDon (364,6 g), Toluol (360 ml), Tetrabutylammoniumiodid (7,6 g) und Natronlauge (50%ig, 480 g) wurden in den Reaktor eingefüllt und unter Rühren (400 UpM) auf 90 °C aufgeheizt. Bei 90 °C Innentemperatur wurde mit der Dosierung von Allylchlorid (306,1 g) begonnen, wobei die Temperatur im Reaktor abfiel und Rückfluss einsetzte. Gesamtdosierdauer: 3 h. Das zweiphasige Reaktionsgemisch wurde dann über Nacht bei 94 °C gerührt. Danach wurde die Reaktionslösung auf RT abgekühlt. Bei 65 °C wurden dabei 500 ml Wasser zugegeben, um den angefallenen Feststoff in der wässrigen Phase zu lösen. Nach der Phasentrennung wurde die organische Phase zweimal mit 500 ml Wasser gewaschen. Danach wurde die organische Phase noch mit 500 g 10%iger Schwefelsäure gewaschen. Die wässrigen Phasen wurden jeweils verworfen.

### Cyclisierung:

213,5 g Allylierungsprodukt werden in einem Destillationskolben mit 5 ml Schwefelsäure versetzt. In der Destillationsapparatur mit 30 cm Füllkörperkolonne (3 mm Metalraschigringe) und Normag-Kolonnenkopf wurde ein Vakuum von 2 mbar angelegt und bei einer Sumpftemperatur von 138-142 °C (Kopftemperatur 108-115 °C) 163,2 g Produkt abdestilliert.

### Beispiel 2:

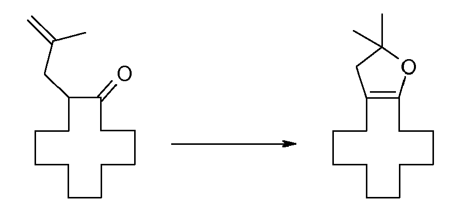

Methallylcyclododecanon wurde ausgehend von CDon und Methallylchlorid, analog wie in Beispiel 1 im Abschnitt Allylierung beschrieben, hergestellt.

### Brönstedt-Säure-Katalyse

In einem 1000 ml Dreihalskolben mit 30 cm Sulzerkolonne und Normag-Kolonnenkopf wurden 1006 g Methallylcyclododecanon vorgelegt und mit 20 g konzentrierter Schwefelsäure versetzt. Es wurde ein Vakuum von 1 mbar angelegt, die Ölbadtemperatur wurde auf 140 °C erhöht. Bei einer Sumpftemperatur von 128-135 °C wurde der Bicyclus langsam aus dem Reaktionsgemisch herausdestilliert (Kopftemperatur 91-96 °C). Insgesamt konnten 866,35 g Produkt aus dem Reaktionsgemisch herausdestilliert werden.

### Lewis-Säure-Katalyse

In einem 500 ml Destillationskolben mit 30 cm Füllkörperkolonne (3 mm Drahtringe) und Normag-Kolonnenkopf wurden 200 g 2-(2-Methallyl-)cyclododecanon vorgelegt und mit 2 g Aluminiumchlorid versetzt. Es wurde ein Vakuum von 2 mbar angelegt, die Ölbadtemperatur wurde langsam auf 175 °C erhöht. Bei einer Sumpftemperatur von 153-156 °C wurde das Produkt aus dem Reaktionsgemisch herausdestilliert (Kopftemperatur 121-123 °C). Insgesamt konnten 140,5 g Produkt aus dem Reaktionsgemisch herausdestilliert werden.

### Beispiel 3:

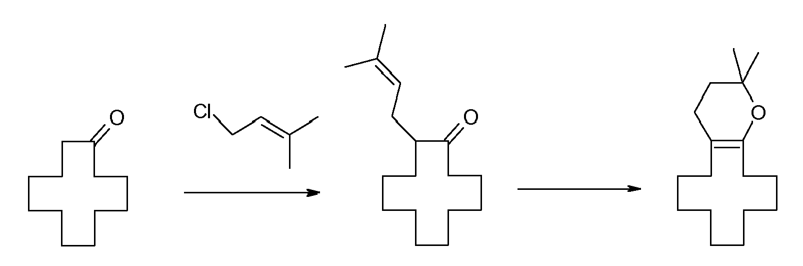

CDon (364,6 g), Toluol (360 ml), Tetrabutylammoniumiodid (7,6 g) und Natronlauge (50%ig, 480 g) wurden in den Reaktor eingefüllt und unter Rühren (400 UpM) auf 90 °C aufgeheizt. Bei 90 °C Innentemperatur wurde mit der Dosierung von 1-Chlor-3-methyl-2-buten (313,7 g) begonnen. Die Temperatur wurde während der gesamten Zugabe bei 90 °C gehalten. Gesamtdosierdauer: 3 h. Das zweiphasige Reaktionsgemisch wurde für 5 h bei 90 °C nachgerührt. Danach wurde die Reaktionslösung auf RT abgekühlt. Bei 65 °C wurden 500 ml Wasser zugegeben, um den angefallenen Feststoff in der wässrigen Phase zu lösen. Nach der Phasentrennung wurde die organische Phase zweimal mit 500 ml Wasser gewaschen. Danach wurde die organische Phase noch mit 500 g 10%iger Schwefelsäure gewaschen. Die wässrigen Phasen wurden jeweils verworfen.

Zu 278 g des Zwischenproduktes wurden 7 g konz. Schwefelsäure gegeben und dann die Lösung in einen 1l Destillationskolben umgefüllt und in einer 70 cm Füllkörperkollonne (3 mm Metalraschigringe) mit Rücklaufteiler bei einer Sumpftemperatur von 185°C einer Kopftemperatur von 130-135 °C einem Druck von 3 mbar und einem Rücklaufverhältnis von 40:1 bis 60:1 destilliert. 191,7 g des Produktes konnten aus der Reaktionsmischung herausdestilliert werden.

### Beispiel 4:

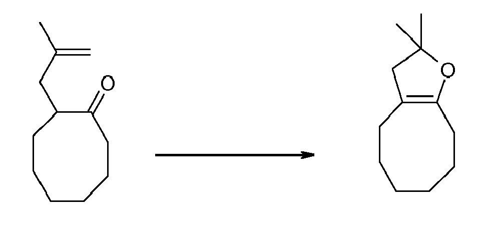

2-Methallylcyclooctanon wurde ausgehend von Cyclooctanon und Methallylchlorid, analog wie in Beispiel 1 im Abschnitt Allylierung beschrieben, hergestellt.

In einem 1 l Destillationskolben mit 30 cm Füllkörperkolonne (3 mm Drahtringe), Normag-Kolonnenkopf und Vakuumregler wurden 302,8 g 2-Methallylcyclooctanon vorgelegt. 3 g konzentrierte Schwefelsäure wurden zugegeben und ein Vakuum von 5 mbar wurde angelegt. Die Ölbadtemperatur wurde langsam auf 130 °C erhöht. Bei einer Sumpftemperatur von 100-105 °C wurde der gebildete Bicyclus langsam aus dem Reaktionsgemisch herausdestilliert. (Kopftemperatur 77 °C). Insgesamt konnten 228,2 g Bicyclus mit einer Reinheit von >96% als Fraktionen erhalten werden.

### Beispiel 5:

3-Methallyl-4-heptanon wurde ausgehend von 4-Heptanon und Methallylchlorid, analog wie in Beispiel 1 im Abschnitt Allylierung beschrieben, hergestellt.

In einem 1 l Destillationskolben mit 30 cm Füllkörperkolonne (3 mm Drahtringe), Normagkolonnenkopf und Vakuumregler wurden 268 g 3-Methallyl-4-heptanon vorgelegt und mit 2,68 g konzentrierter Schwefelsäure versetzt. Ein Vakuum von 20 mbar wurde angelegt und die Ölbadtemperatur auf 110 °C erhöht. Bei einer Sumpftemperatur von 83-88 °C wurde das gebildete Dihydrofuran aus dem Reaktionsgemisch herausdestilliert (Kopftemperatur 67-69 °C). Insgesamt konnten 208,1 g Produkt erhalten werden.

## Patentansprüche

1. Verfahren zur Herstellung von cyclischen Enolethern der Formeln (I) und/oder (II) 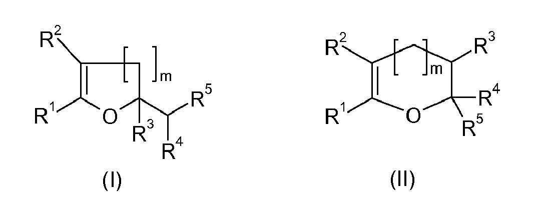 durch Cyclisierung einer Ausgangsverbindung der Formel (III) 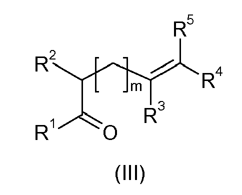 worin
m gleich null (0), eins (1) oder zwei (2) ist,
R¹ ein organischer Rest mit 1 bis 20 Kohlenstoffatomen ist,
R² Wasserstoff oder ein organischer Rest mit 1 bis 20 Kohlenstoffatomen ist, oder die Reste R¹ und R² zusammen mit den sie verbindenden Atomen ein mono- oder polycyclisches, substituiertes oder unsubstituiertes Ringsystem mit 3 bis 20 Kohlenstoffatomen bilden, das auch Heteroatome, ausgewählt aus der Gruppe bestehend aus den Elementen Si, N, P, O, und S enthalten kann,
R³ Wasserstoff oder ein organischer Rest mit 1 bis 20 Kohlenstoffatomen ist,
R⁴ Wasserstoff oder ein organischer Rest mit 1 bis 20 Kohlenstoffatomen ist, und
R⁵ Wasserstoff oder ein organischer Rest mit 1 bis 20 Kohlenstoffatomen ist,
in Gegenwart einer Brönstedt- oder Lewis-Säure, wobei die Reaktion als Reaktivdestillation durchgeführt wird, wobei die gebildeten cyclischen Enolether der Formeln (I) und/oder (II) destillativ aus dem Reaktionsgemisch von der Ausgangsverbindung der Formel (III) abgetrennt werden.

2. Verfahren gemäß Anspruch 1, wobei das molare Verhältnis der Brönstedt- oder Lewis-Säure zur Verbindung der Formel (III) nicht größer als 1 ist.

3. Verfahren gemäß Anspruch 1 oder 2, wobei die Cyclisierung in Gegenwart einer Brönstedtsäure mit einem pKₛ-Wert von kleiner 5 durchgeführt wird.

4. Verfahren gemäß einem der Ansprüche 1 bis 3, worin in den Formeln (I), (II) und (III)
m gleich null (0), eins (1) oder zwei (2) ist,
R¹ ein C₁-C₁₀-Alkylrest ist,
R² ein C₁-C₁₀-Alkylrest ist,
oder die Reste R¹ und R² zusammen für eine zweibindige Gruppe -(CH₂)ₓ- stehen, wobei x eine ganze Zahl von 3 bis 12 bedeutet,
R³ Wasserstoff oder Methyl ist,
R⁴ Wasserstoff oder ein Methyl ist, und
R⁵ Wasserstoff oder Methyl ist.

5. Verfahren gemäß einem der Ansprüche 1 bis 3, wobei die Ausgangsverbindung der Formel (III), die für eine Verbindung der Formel (IIIa) oder eine Verbindung der Formel (IIIb) steht, 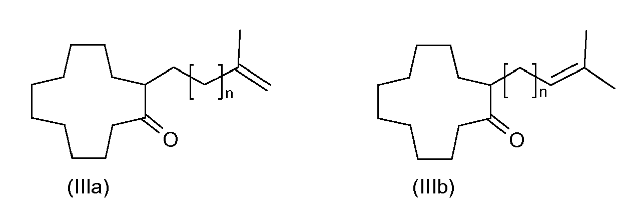 zu dem entsprechenden cyclischen Enolether der Formel (IIa) umgesetzt wird, 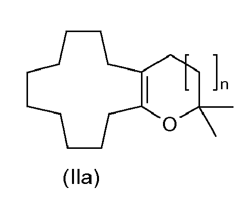 worin in den Formeln (IIa), (IIIa) und (IIIb)
n gleich null (0) oder eins (1) ist.
